# EUROPÄISCHE PATENTSCHRIFT

(11) **EP 2 349 387 B1**
(45) Veröffentlichungstag und Bekanntmachung des Hinweises auf die Patenterteilung: **29.08.2012**
(21) Anmeldenummer: 09759677.9
(22) Anmeldetag: 19.11.2009
(51) Int. Cl.: A61M 1/14, A61J 1/10

(54) **EINWEGBEUTEL UMFASSEND EINE MEHRSCHICHTFOLIE**
DISPOSABLE BAG COMPRISING A MULTILAYER FILM
SACHET A USAGE UNIQUE COMPORTANT UNE FEUILLE MULTICOUCHES

(30) Priorität: 20.11.2008 DE 102008058272
(43) Veröffentlichungstag der Anmeldung: 03.08.2011
(73) Patentinhaber: Fresenius Medical Care Deutschland GmbH, 61352 Bad Homburg v.d.H. (DE)
(72) Erfinder: HERRENBAUER, Michael, 61267 Neu-Anspach (DE); WEIS, Manfred, 66606 St. Wendel (DE); KUGELMANN, Franz, 66606 St. Wendel (DE)
(74) Vertreter: Stolmár Scheele & Partner
(86) Internationale Anmeldenummer: PCT/EP2009/008248
(87) Internationale Veröffentlichungsnummer: WO 2010/057643

(56) Entgegenhaltungen:
- DE-A1- 2 649 657
- DE-C1- 19 959 230
- US-A1- 2004 146 671

## Beschreibung

Die vorliegende Erfindung betrifft einen Einwegbeutel zur Aufnahme einer Flüssigkeit, der eine Mehrschichtfolie umfasst, sowie eine Vorrichtung, die eine Stützform für den erfindungsgemäßen Einwegbeutel umfasst, in der der Einwegbeutel angeordnet ist. Zudem betrifft die vorliegende Erfindung ein Verfahren zur Herstellung des Einwegbeutels.

Hämodialysegeräte sind in verschiedenen Ausführungen bekannt. Der Stoffaustausch zwischen dem Blut und der Dialysierflüssigkeit findet in einem Dialysator statt, der einen ersten Strömungsweg für das Blut und einen zweiten Strömungsweg für die Dialysierflüssigkeit aufweist, wobei beide Strömungswege durch eine semipermeable Membran voneinander getrennt sind. Der erste Strömungsweg ist Bestandteil eines extrakorporalen Blutkreislaufs mit einer Zuführleitung und einer Rückführleitung für das Blut sowie ggfs. einer den Blutfluss unterstützenden Pumpe. Der zweite Strömungsweg ist mit Einrichtungen zum Zuführen und Abführen der Dialysierflüssigkeit verbunden.

Behälter der aus dem Stand der Technik bekannten Hämodialysegeräte bestehen oft aus Glas, das anderen Werkstoffen wegen der porenfreien Oberfläche hygienisch und bakteriologisch überlegen ist. Darüber hinaus ist Glas weitgehend resistent gegen in Betracht kommende Chemikalien, gut zu reinigen und physiologisch unbedenklich.

Bekannte Hämodialysegeräte zeichnen sich durch einen relativ einfachen Aufbau aus. Nachteilig ist jedoch, dass der Glasbehälter verhältnismäßig teuer in der Herstellung ist. Desweiteren erweist sich die vor der Dialysebehandlung erforderliche Desinfektion des Glasbehälters als nachteilig, da oft eine schnelle Wiederverwendung des Gerätes erforderlich ist. Deshalb wäre es wünschenswert eine Hämodialysegerät bereitzustellen, in dem das Gerät während der erforderlichen Desinfektion des Behälters, der die Dialyseflüssigkeiten enthält, weiter betrieben werden kann.

Um diese Aufgabe zu lösen, schlägt das US 4,767,526 ein Dialysegerät vor, bei dem ein Behältertank, in dem die Dialysierflüssigkeit bereitgestellt wird, mit einem Beutel ausgekleidet ist, der nach dem Gebrauch verworfen wird. Dies hat den Vorteil, dass der Behältertank nach Entfernen des Beutels schnell wieder zur Verfügung steht, und der Behältertank an sich nicht desinfiziert werden muss.

Das DE 198 25 158 C1 beschreibt einen großvolumigen Beutel, der aus zwei kegelförmigen Folienblättern zusammengeschweißt wird. Um eine sicherere Trennung von frischer Dialysierflüssigkeit und verbrauchtem Dialysat zu gewährleisten, kann eine zusätzliche Trennfolie in den Beutel eingeschweißt sein.

Das EP 1 235 601 B1 beschreibt einen Beutel für die Dialysat-Bereitstellung in einem Tank. Um diesen Beutel in den Behälter einzubringen bedient man sich eines speziellen Faltmechanismus, aus dem der Beutel aus einem handlichen Format zum Beutel entfaltet wird.

Die WO 83/02061 beschreibt einen Beutel aus PVC (Polyvinylchlorid) mit mehreren Kammern für die Peritonealdialyse. Dieser Beutel wird für die getrennte Lagerung von Dialyselösungsbestandteilen, die separat entnommen werden und erst vermischt werden sollen, wenn sie dem Patienten verabreicht werden, verwendet. Nachteilig an diesem Beutel ist, dass er aus PVC besteht.

Die Verwendung der zuvor genannten Beutel in Dialysegeräten löst zwar die Aufgabe, dass der Tank selbst nicht mehr zeitaufwendig desinfiziert werden muss, jedoch sind diese Beutel nachteilig hinsichtlich ihrer Größe und der damit verbundenen umständlichen Handhabung.

Diese relativ großflächigen Beutel können zu Problemen im Klinikalltag führen, in dem ein Beutel in der Regel von teilweise wenig geschultem Klinikpersonal schnell ausgetauscht werden muss. So birgt die große Fläche der Beutel die Gefahr von fehlerhafter Bedienung bei Einführen des Beutels in das Tankinnere. Dadurch kann der Beutel fehlerhaft zerknittert sein, so dass er sich beim Befüllen mit dem Dialysat nicht vollständig entfaltet.

Zudem können auch bei der Entnahme von Flüssigkeit Restmengen aufgrund von Faltenbildung nicht zu entnehmen sein. Um frisches bzw. verbrauchtes Dialysat ablassen bzw. zuführen zu können, besitzen Dialysebeutel Ablass- bzw. Zuführöffnungen. Bei einer fehlerhaften Faltung bzw. Zerknittern des Beutels bei zu schneller Einbringung in den Tankbehälter können sich zudem Falten im Beutel so legen, dass die essentiellen Öffnungen teilweise oder ganz verschlossen sind.

Versuche, Einwegbeutel bereits in einer speziellen (Vor)Faltung in die Vorrichtung einzubringen, die eine fehlerhafte Entfaltung verhindern sollte, waren ebenfalls nicht erfolgreich. Zudem ließ sich bei den großräumigen Einwegbeuteln bisher keine Faltung ermitteln, die Probleme bei der Entfaltung vollständig verhinderte.

Ein weiteres Problem der Einwegbeutel des Standes der Technik ist ihre mangelhafte Stabilität, besonders, wenn sie große Volumina, wie sie insbesondere für die vorliegende Erfindung von Bedeutung sind, aufnehmen müssen. Dies ist insbesondere darauf zurückzuführen, dass das Gewicht bzw. der Druck, der auf den Schweißnähten lastet mit Zunahme der Füllmenge stark ansteigt. Ein weiterer Nachteil der bekannten Beutel ist, dass aufgrund ihrer Größe ein hoher Materialeinsatz erforderlich ist, was wiederum ein hohes Abfallvolumen mit sich bringt.

Es war deshalb eine Aufgabe der Erfindung einen Einwegbeutel bzw. eine Vorrichtung, die zum Befüllen mit Dialysierflüssigkeit geeignet ist, bereitzustellen, der die zeitraubende Desinfektion des Tankbehälters des Dialysegeräts nicht erforderlich macht, eine sichere, schnelle und faltenbildungsfreie Handhabung beim Einbringen des Einwegbeutels in den Tankbehälter des Dialysegeräts im Klinikalltag ermöglicht, wenig Materialeinsatz erfordert und eine hohe Stabilität des Beutels, d.h. hohe Transportsicherheit beim Ein- und Ausbringen des/der befüllten Einwegbeutels/Vorrichtung aus dem Tankbehälter mit sich bringt.

Die Aufgabe wird erfindungsgemäß gelöst durch einen Einwegbeutel gemäß dem Anspruch 1. Der Einwegbeutel besteht aus einer Mehrschichtfolie zur Aufnahme einer Dialysier-Flüssigkeit, wobei die Mehrschichtfolie in Längsrichtung der Extrusion der Folie eine Reißdehnung von 250 % bis 850 %, bevorzugt 400 % bis 800 %, mehr bevorzugt 500 % bis 750 % und am meisten bevorzugt 600 % bis 700 %, und in Querrichtung der Extrusion der Folie von 300 % bis 1050 %, bevorzugt 450 % bis 1000 %, mehr bevorzugt 600 % bis 900 % und am meisten bevorzugt 700 % bis 800 % aufweist.

Unter Reißdehnung oder Bruchdehnung versteht man das prozentuale Verhältnis der Längenänderung ΔL (beim Bruch) zur Ausgangslänge. Sie drückt die Fähigkeit eines Werkstoffes aus, Formänderungen ohne Rissbildung zu folgen. Die Reißdehnung wird bei Zugversuch gemäß DIN 53455 gemessen.

Eine große Fähigkeit zur Längenänderung in Längsrichtung der Extrusion der Folie im oben genannten Bereich hat den erfindungsgemäßen Vorteil, dass der Beutel, während er mit Dialysat (verbraucht oder frisch) be- oder entfüllt wird, einer Volumenänderung unterliegt, ohne dass er vor den angegebenen Obergrenzen Risse bildet. Das bringt den weiteren Vorteil mit sich, dass im unbefüllten Zustand nur wenig Material erforderlich ist, jedoch trotzdem ein großes Fassungsvolumen im befüllten Zustand vorliegt. Dadurch kann ein Produkt bereit gestellt werden, das nur ein geringes Abfallvolumen mit sich bringt. Dies ist insbesondere unter Umweltschutzaspekten ein großer Vorteil.

Unter "Einwegbeutel" wird jeder Gegenstand verstanden, der die Aufnahme einer Flüssigkeit, eines Feststoffes und/oder eines Gases für einen bestimmten Zeitraum ermöglicht. Zudem wird unter einem Einwegbeutel im Rahmen der vorliegenden Erfindung jeder Gegenstand verstanden, der für die mindestens einmalige Verwendung in der beabsichtigten Anwendung geeignet ist.

Unter dem Begriff "Mehrschichtfolie" wird in der vorliegenden Erfindung eine Folie verstanden, die aus zwei oder mehr Schichten verschiedenen oder gleichen Materials besteht, die adhäsiv miteinander verbunden sind.

Dabei ist es im Rahmen der vorliegenden Erfindung bevorzugt, dass die Mehrschichtfolie aus 2 bis 10 Schichten aufgebaut ist, wobei ein Aufbau aus 2 bis 5 Schichten bevorzugter und ein Aufbau aus 3 oder 4 Schichten besonders bevorzugt ist. Die Mehrschichtfolie kann gemäß jedem Verfahren hergestellt werden, das dem Fachmann als für den erfindungsgemäßen Zweck geeignet bekannt ist.

In einer weiteren Ausführungsform der vorliegenden Erfindung ist es bevorzugt, dass die Mehrschichtfolie des erfindungsgemäßen Einwegbeutels in Längsrichtung eine Extrusion der Folie eine Reißfestigkeit von 300 N/mm² bis 350 N/mm², bevorzugt 310 N/mm² bis 340 N/mm² und mehr bevorzugt 320 N/mm² bis 330 N/mm², und in Querrichtung der Extrusion der Folie von 220 N/mm² bis 270 N/mm², bevorzugt 230 N/mm² bis 260 N/mm² und mehr bevorzugt 240 N/mm² bis 250 kp/cm² aufweist.

Unter "Reißfestigkeit" versteht man die Zugspannung, die auf einen Gegenstand im Augenblick des Reißens ausgeübt wird. Die Reißfestigkeit wird im Zugversuch gemäß der DIN 53455 gemessen. Eine Reißfestigkeit unterhalb der oben genannten Untergrenze ist nachteilig, da der Beutel sonst durch Überdehnung früh reißt. Oberhalb der angegebenen Obergrenze ist der Beutel zwar sehr reißfest, jedoch nicht ausreichend dehnbar.

In einer weiteren Ausführungsform der vorliegenden Erfindung hat die Mehrschichtfolie des erfindungsgemäßen Einwegbeutels vorzugsweise eine Querdehnzahl µ im gummielastischen Zustand von 0,45 bis 0,55, mehr bevorzugt 0,47 bis 0,53 und am meisten bevorzugt 0,49 bis 0,51.

Die Querdehnzahl, auch Poissonzahl genannt, ist definiert als Verhältnis aus relativer Dickenänderung Δd/d zur relativen Längenänderung Δ1/1 bei Einwirkung einer äußeren Kraft oder Spannung.

Eine weitere erfindungsgemäße Ausführungsform ist ein Einwegbeutel, bei dem die Mehrschichtfolie durch eine Kraft von vorzugsweise 45 N bis 60 N, mehr bevorzugt 48 N bis 62 N, am meisten bevorzugt 52 N bis 58 N um bis zu 500 % gedehnt werden kann. Um die Dehnbarkeit zu messen wird ein Gewicht, das einer bestimmten Kraft in N entspricht, gleichmäßig an eine 15 mm breite Folie angebracht und die Längenänderung gemessen.

Eine hohe Dehnbarkeit hat den Vorteil, dass der Beutel im unbefüllten Zustand eine geringe Größe aufweist und somit einfach handhabbar ist. Zudem ist der Materialbedarf durch die starke Dehnbarkeit des Materials gering. Somit wird auch eine einfachere Fertigung und Verpackung des Materials ermöglicht.

Eine besonders bevorzugte Ausführungsform der vorliegenden Erfindung ist auch ein Einwegbeutel bei dem das Verhältnis der äußeren Oberfläche des Einwegbeutels im maximal befüllten Zustand zu der äußeren Oberfläche im unbefüllten Zustand im Bereich von vorzugsweise 5/1 größer ≥ 2/1, mehr bevorzugt ≥ 5/1.

Typische Obergrenzen liegen bei ca. 8/1 bis 12/1 z.B. 10/1 oder 9/1. Auch höhere Verhältnisse sind jedoch erfindungsgemäß vorgesehen.

Unter "äußerer Oberfläche" versteht man die Fläche des Beutels, die mit der Umgebung (Luft) im befüllten sowie unbefüllten Zustand in Kontakt treten kann. Der Begriff "maximal befüllter Zustand" wird durch die maximale Größe des Beutels, bei der der Beutel gerade noch keine Risse bildet und folglich noch nicht reißt, beschrieben.

Unter "unbefüllter Zustand" versteht den Zustand des Beutels in dem das Innere des Beutels im Wesentlichen nicht durch Materie jeglicher Art ausgefüllt ist, d.h. im Wesentlichen keinen Raum einnimmt.

Die Eigenschaft der Oberflächenzunahme in Abhängigkeit von der Befüllmenge gewährleistet, dass die Mehrschichtfolie des Beutels bei Befüllung immer unter Druck steht, so dass bei zunehmender Befüllung dieser Druck steigt und eventuelle Falten in der Mehrschichtfolie, die im unbefüllten Zustand vorliegen können, immer mehr verschwinden. Dies hat den erfindungsgemäßen Vorteil, dass eine faltenfreie Einbringung des Einwegbeutels in einen Tankbehälter einer medizinischen Apparatur, insbesondere Dialysegerät, gewährleistet ist. Somit ist auch die vollständige Entnahme der Flüssigkeit aus dem Beutel gewährleistet.

Gemäß der vorliegenden Erfindung ist das Fassungsvolumen des Einwegbeutels im maximal befüllten Zustand zu dem Fassungsvolumen im Zustand, in dem die Mehrschichtfolie dehnungsfrei vorliegt, vorzugsweise ≥ 3/1, bevorzugt ≥ 5/1 ist. Typische nicht einschränkende Bereiche betragen dabei 3/1 bis 12/1, mehr bevorzugt 5/1 bis 11/1, noch mehr bevorzugt 7/1 bis 10/1 und am meisten bevorzugt 8/1 bis 9/1.

Andere höhere Obergrenzen sind aber auch erfindungsgemäß möglich.

Unter "Fassungsvolumem im Zustand, in dem die Mehrschichtfolie dehnungsfrei vorliegt" versteht man das Volumen, das in den Beutel eingefüllt werden kann, ohne dass eine Dehnung der Mehrschichtfolie stattfindet.

In einer weiteren Ausführungsform der vorliegenden Erfindung hat der Einwegbeutel der oben genannten Ausführungsformen vorzugsweise ein Fassungsvolumen im maximal befüllten Zustand im Bereich von 30 L bis 120 L, besonders bevorzugt 40 L bis 110 L, noch mehr bevorzugt 50 L bis 100 L, weiter bevorzugt 60 L bis 90 L und am meisten bevorzugt 70 L bis 80 L aufweist. Der Einwegbeutel der vorliegenden Erfindung kann eine Kammer oder mehr als eine (mehrere) Kammern enthalten. Unter dem Begriff "mehrere" wird das Vorliegen von mindestens 2 Kammern verstanden. Der Einwegbeutel kann jedoch über so viele Kammern verfügen, wie es dem Fachmann für den jeweiligen Verwendungszweck geeignet erscheint.

Der Einwegbeutel enthält bevorzugt 1 bis 10 Kammern, bevorzugter 2 bis 6 Kammern, mehr bevorzugt 2 bis 4 und am meisten bevorzugt 2 oder 3 Kammern.

Enthält der Einwegbeutel nur eine Kammer, so können auch zwei Einwegbeutel im Dialysegerät verwendet werden, einer für die Bereitstellung von frischem Dialysat und einer, in den verbrauchtes Dialysat rückgeführt wird. Enthält der Einwegbeutel jedoch mehrere Kammern, so wird vorzugsweise eine der Kammern für die Bereitstellung von frischem Dialysat verwendet, und eine Kammer zum Auffangen von verbrauchtem Dialysat.

Ein solcher Einsatz des/der erfindungsgemäßen Einwegbeutel(s) hat den Vorteil, dass eine Trennung von frischem und verbrauchtem Füllmaterial möglich ist und somit keine Kreuzkontamination auftritt. Zudem muss am Verwendungsort kein Wasserablauf vorhanden sein, da durch den Einsatz eines Beutels mit mehreren Kammern oder von zwei oder mehr Beuteln mit einer Kammer das verbrauchte Dialysat in einer Kammer aufgefangen werden kann. Die einzelnen Kammern werden dabei bevorzugt ebenfalls aus Teilen der Mehrschichtfolie, wie sie nachstehend näher definiert ist, gefertigt.

Im Rahmen der vorliegenden Erfindung ist eine bevorzugte Ausführungsform des Einwegbeutels mit Kammern ausgestattet, die in Längsrichtung des Einwegbeutels angeordnet sind.

Die einzelnen Kammern können dabei jeweils über eine oder mehrere Zuführ- und Ablassleitung(en) verfügen, wobei es bevorzugt ist, dass - besonders im Fall eines Einwegbeutels mit 2 Kammern - zumindest eine Kammer über eine Zuführleitung und die andere Kammer über eine Ablassleitung verfügt. Es kann jedoch auch jede einzelne Kammer über eine Zuführ- und Ablassleitung verfügen.

Die Zuführ- und Ablassleitung(en) kann/können mit dem Einwegbeutel fest verschweißt oder aber über ein Dichtungssystem fest oder abnehmbar verbunden sein. Die Schlauchanschlüsse an den Einwegbeutel können konventionelle Anschlussstücke sein. Entscheidend ist, dass der Einwegbeutel schnell an die Zuführ- und Ablassleitung(en) anschließbar bzw. zum Austausch des Einwegbeutels wieder abnehmbar ist. Es ist aber auch möglich, dass das Zuführ- bzw. Ablassleitungs-System einstückig mit dem Einwegbeutel ausgebildet ist.

In der erfindungsgemäßen Ausführungsform umfasst die Mehrschichtfolie eines Einwegbeutels eine Schicht des Typs (A), die mehrere Komponenten enthält, wobei eine der Komponenten aus der Gruppe ausgewählt ist, die aus Styrol-Isopren Block-Copolymeren, Styrol-Ethylen-Butylen-Styrol-Block-Copolymeren, Styrol-Ethylen-Butylen Block-Copolymeren, Styrol-Ethylen-Propylen Block-Copolymeren und Mischungen davon besteht, und eine weitere Komponente aus der Gruppe ausgewählt ist, die aus Polyethylen und einem statistischen Copolymer besteht, das Ethylen-Einheiten umfasst.

Besonders bevorzugt ist die weitere Komponente in der Schicht des Typs (A) ein statistisches Copolymer, das sich aus Ethylen- und Octen-Einheiten zusammensetzt.

Der Anteil der einen Komponente in der Schicht des Typs (A) kann dabei im Bereich von 35 % bis 99,99 % liegen, bevorzugt 50 % bis 99.9 %, mehr bevorzugt 55 % bis 99 %, noch mehr bevorzugt 60 % bis 95 % und am meisten bevorzugt 60 % bis 90 %, bezogen auf die gesamte Zusammensetzung der Schicht des Typs (A).

Die weiteren Komponente in der Schicht des Typs (A) kann somit im Bereich von 0,01 % bis 65 %, bevorzugter bei 0,1 % bis 50 %, mehr bevorzugt 1 % bis 45 %, noch mehr bevorzugt 5 bis 40 % und am meisten bevorzugt bei 10 % bis 40 % liegen, bezogen auf die gesamte Zusammensetzung der Schicht des Typs (A).

Erfindungsgemäß umfasst die Mehrschichtfolie zudem eine oder mehrere Schichte(n) des Typs (B), die unabhängig voneinander mehrere Komponenten enthält, wobei eine der Komponenten aus der Gruppe ausgewählt ist, die aus Styrol-Ethylen-Butylen Block-Copolymere (SEB), Styrol-Ethylen-Butylen-Styrol-Block-Copolymeren (SEBS), Styrol-Ethylen-Propylen Block-Copolymere (SEP), Styrol-Isopren-Styrol Block-Copolymere (SIS) und Mischungen davon besteht, und eine weitere Komponente aus der Gruppe ausgewählt ist, die aus Polypropylen und einem statistischen Copolymer, das Propylen-Einheiten umfasst, besteht.

Besonders bevorzugt ist, dass die weitere Komponente in der Schicht des Typs (B) sich aus einem statistisches Copolymer, das Propylen- und Ethylen-Einheiten umfasst, zusammensetzt.

Folien mit einer Schicht des Typs (B) sind hinsichtlich ihrer hohen Dehnbarkeit vorteilhaft. Der Anteil der einen Komponente in der Schicht des Typs (B) kann dabei im Bereich von 35 % bis 99,99 %, bevorzugt 50 % bis 99.9 %, mehr bevorzugt 55 % bis 99 %, noch mehr bevorzugt 60 % bis 95 % und am meisten bevorzugt 60 % bis 90 % sein, bezogen auf die gesamte Zusammensetzung der Schicht des Typs (A).

Die weitere Komponente in der Schicht des Typs (B) kann somit im Bereich von 0,01 % bis 65 %, bevorzugter bei 0,1 % bis 50 %, mehr bevorzugt 1 % bis 45 %, noch mehr bevorzugt 5 bis 40 % und am meisten bevorzugt bei 10 % bis 40 % liegen, bezogen auf die gesamte Zusammensetzung der Schicht des Typs (B).

In einer ganz besonders bevorzugten Ausführungsform der vorliegenden Erfindung ist die Mehrschichtfolie vorzugsweise aus einem Laminat aus 3 Schichten aufgebaut. Die mittlere Schicht ist vorzugsweise eine Schicht des oben genannten Typs (A). Die beiden äußeren Schichten, die die Schicht des Typs (A) umgeben, sind vorzugsweise Schichten des Typs (B). Diese äußeren Schichten des Typs (B) können eine gleiche oder voneinander verschiedene Zusammensetzung(en) haben.

Diese Folien sind besonders bevorzugt, da sie eine gleichmäßige Dehnung des erfindungsgemäßen Einwegbeutels über die gesamte Befüllung hin gewährleisten.

Unter der Bezeichnung "Polyethylen" wird im Rahmen der vorliegenden Erfindung ein durch Polymerisation von Ethylen hergestellter thermoplastischer Kunststoff mit der vereinfachten Ketten-Strukturformel (C₂H₂)ₙ verstanden. Ein Copolymer, das Ethylen-Einheiten umfasst, ist ein durch Copolymerisation von Ethylen mit einem beliebigen weiteren Olefin hergestelltes Polymer. Polyethylen und ein Copolymer, das Ethylen-Einheiten umfasst, gehören zur Gruppe der Polyolefine.

Das sogenannte "weitere Olefin" ist vorzugsweise ein α-Olefin, das 3 bis 20 Kohlenstoffatome umfasst. Beispiele für das beliebige weitere Olefin sind Propylen, Buten, Penten, Hexen, Hepten und Octen, wobei Octen besonders bevorzugt ist.

Unter der Bezeichnung "Polypropylen" wird im Rahmen der vorliegenden Erfindung ein durch Polymerisation von Propylen hergestellter thermoplastischer Kunststoff mit der vereinfachten Ketten-Strukturformel (C₃H₆)ₙ verstanden. Ein Copolymer, das Propylen-Einheiten umfasst, ist ein durch Copolymerisation von Propylen mit einem beliebigen weiteren Olefin hergestelltes Polymer. Polypropylen und ein Copolymer, das Propylen-Einheiten umfasst, gehören zur Gruppe der Polyolefine.

Das sogenannte "weitere Olefin" ist vorzugsweise ein α-Olefin, das 2 bzw. 4 bis 20 Kohlenstoffatome umfasst. Beispiele für das beliebige weitere Olefine sind Ethylen, Buten, Penten, Hexen, Hepten und Octen, wobei Ethylen besonders bevorzugt ist.

Bekannte Handelsnamen diverser Polyolefine sind: Alathon^{®}, Dyneema^{®}, Hostalen^{®}, Lupolen^{®}, Polythen^{®}, Spectra^{®}, Trolen^{®}, Vestolen^{®}.

Allgemein unterscheidet man zwischen:
- PE-HD (HDPE): schwach verzweigte Polymerketten, daher hohe Dichte zwischen 0,94 g/cm³ und 0,97 g/cm³, ("HD" steht für "high density").
- PE-LD (LDPE): stark verzweigte Polymerketten, daher geringe Dichte zwischen 0,915 g/cm³ und 0,935 g/cm³, ("LD" steht für "low density").
- PE-LLD (LLDPE): lineares Polyethylen niederer Dichte, dessen Polymermolekül nur kurze Verzweigungen aufweist. Diese Verzweigungen werden durch Copolymerisation von Ethen und höheren α-Olefinen (typischerweise Buten, Hexen oder Octen) hergestellt ("LLD" steht für "linear low density").
- PE-HMW: hochmolekulares Polyethylen. Die Polymerketten sind länger als bei PE-HD, PE-LD oder PE-LLD, die mittlere Molmasse liegt bei 500 kg/mol bis 1000 kg/mol.
- PE-UHMW: ultrahochmolekulares Polyethylen mit einer mittleren Molmasse von bis zu 6000 kg/mol und einer Dichte von 0,93 g/cm³ bis 0,94 g/cm³ ("UHMW" steht für "ultra high molecular weight").

Die oben genannten Copolymere, die zumindest Ethylen- und Propylen-Einheiten umfassen, sind vorzugsweise statistische Copolymere. Ein statistisches Copolymer ist ein Copolymer, in dem die mindestens zwei verschiedenen Monomer-Einheiten aus denen das Polymer sich zusammensetzt in einer zufälligen Reihenfolge copolymerisiert vorliegen. Unter den oben genannten Block-Copolymeren versteht man Polymere, bei denen die Monomere nicht statistisch in die Kette eingebaut sind, sondern in denen homopolymere Kettenabschnitte miteinander verknüpft sind. Sie stellen ein Mittel aus einem Copolymerisat und einer Polymermischung dar und bieten die Möglichkeit zur Verbesserung von Polymeren ohne Änderung der Monomerzusammensetzung.

Bevorzugte Beispiele solcher Blockpolymere schließen die folgenden ein: gemischtes Block-Copolymere aus Ethylen, Propylen, alpha-Olefinen oder Isopren und Styrol (sogenannte olefinische Styrolblockcopolymere) bzw. Dien-Styrolblockcopolymere wie z.B. SEBS, SEPS, SBS, SEB, SEP und SIS etc.

Als Elastomere in der Schicht der Typen (A) und (B) können alternativ auch vorzugsweise elastomere Styrol-Butadien- oder Styrol-Isopren-Blockcopolymere, vorzugsweise mit einem Dien-Anteil von über 50 Gew.-%, als auch beispielsweise harzartige Styrol-Butadien- und Styrol-Isopren-Blockcopolymere, vorzugsweise mit einem Dienanteil von unter 50 Gew.-%, bezogen auf das gesamte Gewicht des Copolymers, verwendet werden.

Verwendet man elastomere Styrol-Dien-Blockcopolymere mit einem Dien-Anteil von über 50 Gew.-%, so liegt deren Anteil im Gemisch bevorzugt zwischen 20 Gew.-% und 40 Gew.-%, kann jedoch, je nach Anforderungen, auch höher oder niedriger sein. Harzartige Styrol-Dien-Blockcopolymere mit Styrol gehalten über 50 Gew.-%, insbesondere solche mit etwa 65 Gew.-% bis 95 Gew.-% Styrol, sollen bevorzugt in einer Menge von 40 Gew.-% bis 60 Gew.-% in der erfindungsgemäßen Formmasse enthalten sein. In einer weiteren Ausführungsform ist es bevorzugt, dass die Einwegfolie frei von Polyvinylchlorid ist.

In einer außerordentlich bevorzugten Ausführungsform der vorliegenden Erfindung ist die Mehrschichtfolie aus 3 Schichten aufgebaut, wobei die erste äußere Schicht 60 Gew.-% Styrol-Ethylen-Butylen-Styrol (SEBS) Copolymer und 40 Gew.-% statistisches Polypropylen-Ethylen Copolymer, die mittlere Schicht 60 Gew.-% Styrol-Ethylen-Butylen-Styrol Block-Copolymer und 40 Gew.-% statistisches Polyethylen-Octen Copolymer und die zweite äußere Schicht 60 Gew.-% Styrol-Ethylen-Butylen-Styrol Block-Copolymer (SEBS) und 40 Gew.-% statistisches Polypropylen-Ethylen Copolymer enthält.

Weiter ist es im Rahmen der vorliegenden Erfindung bevorzugt, dass die Mehrschichtfolie hitzesterilisierbar ist. Es ist dabei möglich, dass nur eine Schicht der Mehrschichtfolie hitzesterilisierbar ist, es können aber auch zwei oder mehr oder aber auch sämtliche Schichten der Mehrschichtfolie hitzesterilisierbar sein. Es ist dabei besonders bevorzugt, dass zumindest die Schicht hitzesterilisierbar ist, die die Innenseite des erfindungsgemäßen Einwegbeutels bildet.

Unter "Hitzesterilisierbarkeit" wird hierbei verstanden, dass die Mehrschichtfolie bei und nach der Behandlung mit Wasserdampf bei einer Temperatur im Bereich von 100 bis 140 °C, bevorzugt 110 bis 130 °C und einem Druck von 1 bis 2 bar sowohl ihre äußere Form als auch ihre mechanischen und physikalisch-chemischen Eigenschaften beibehält.

Die Dicke der Mehrschichtfolie liegt bevorzugt in einem Bereich von 1 µm bis 1000 µm, weiter bevorzugt von 5 µm bis 800 µm, besonders bevorzugt von 8 µm bis 500 µm und am meisten bevorzugt von 10 µm bis 150 µm. Dabei ist es möglich, dass die einzelnen Schichten der Mehrschichtfolie jeweils die gleiche Dicke oder unterschiedliche Dicken aufweisen. Besonders bevorzugt ist es, im Fall einer 3- oder mehrschichtigen Mehrschichtfolie, dass die beiden die Außenseiten bildenden Schichten eine Dicke von ≤ 40 µm, bevorzugt ≤ 20 µm, aufweisen.

Die innere Schicht einer 3- oder mehrschichtigen Folie kann bevorzugt eine Dicke von ≥ 500 µm, bevorzugter von ≥ 300 µm und am meisten bevorzugt von ≥ 100 µm, aufweisen, wodurch eine gute Stabilität und Elastizität erreicht wird. Dadurch wird eine hohe Flexibilität und Lastwechselfestigkeit erreicht. Gemäß einer bevorzugten Ausgestaltung der vorliegenden Erfindung weist mindestens eine Schicht der Mehrschichtfolie ferner Additive und Verunreinigungen, wie z.B. Katalysatorreste, in einem Gehalt unter 5000 ppm und unter 200 ppm auf.

Weiter ist es bevorzugt, dass im Fall einer 3- oder mehrschichtigen Folie die jeweils äußeren Schichten von hitzesterilisierbaren Schichten gebildet werden. Daraus ergibt sich der Vorteil, dass es bei der Hitzesterilisation weder zu einem Verkleben der Innenseiten des Einwegbeutels noch zu einem Verkleben der Außenseiten des Einwegbeutels mit anderen Bauteilen kommen kann.

Ferner ist es bevorzugt, dass zumindest eine Schicht der Mehrschichtfolie siegelbar, bevorzugt heißsiegelbar ist. Die Siegeltemperatur liegt dabei vorzugsweise im Bereich von 160°C bis 230°C, bevorzugter im Bereich von 180°C bis 210°C.

Zudem ist es bevorzugt, dass die Mehrschichtfolie biokompatibel und besonders bevorzugt hämokompatibel ist.

Desweiteren ist es bevorzugt, dass die Mehrschichtfolie eine transparente Folie ist.

Weiter ist die Mehrschichtfolie bevorzugt frei von Gleitmitteln, Weichmachern, Antiblockmitteln, Antistatika sowie Füllstoffen.

Außerdem betrifft die vorliegende Erfindung eine Vorrichtung, die eine Stützapparatur umfasst, in der ein erfindungsgemäßer Einwegbeutel angeordnet ist. Die Stützapparatur ist vorzugsweise so beschaffen, dass sie den Einwegbeutel an den Stellen stützt/stabilisiert, an denen Schweißstellen als erstes zu reißen drohen. Damit wird die Aufgabe gelöst, den Einwegbeutel schnell und sicher aus dem Tankbehälter zu entfernen, und die Kraft an den Schwachstellen des Beutels nach Befüllung abzufangen. Auf diese Weise ist es somit möglich, einen elastischen Behälter ohne die oben genannten Probleme, wie Faltenbildung, fachgerecht und rasch in einen Tankbehälter einzubringen und herauszunehmen.

Die Stützapparatur zur Aufnahme des erfindungsgemäßen Einwegbeutels umfasst vorzugsweise eine Haltevorrichtung, die bevorzugt so beschaffen ist, dass der Einwegbeutel daran im befüllten wie auch unbefüllten Zustand befestigt werden kann. Weiter muss die Stützapparatur groß genug sein, um den Einwegbeutel auch in einem der Anwendung entsprechenden ausgedehnten Volumen aufnehmen zu können, d.h. mindestens das oben genannte maximale Fassungsvermögen des Einwegbeutels aufweisen. Bevorzugt verfügt die Stützapparatur über eine als formgebende Schale ausgebildete Aufnahmeeinheit. Die formgebende Schale weist eine mittlere zylindrische Anlagefläche auf, an die sich eine nach außen gewölbte obere Anlagefläche und eine nach außen gewölbte untere Anlagefläche anschließen.

Die Formen der vorzugsweise gefäßartigen Stützapparatur sind keiner Limitierung unterworfen. Es können ganz unterschiedlich gewölbte und anders ausgeformte Innenräume der Stützapparatur verwendet werden, sofern dies aus technischen Gründen oder ästhetischen Gründen notwendig ist. Eine sichere Lagerung des Dialysats wird durch den elastischen Beutel in jedem Fall gewährleistet.

Vorteil einer solchen formgebenden Schale ist, dass der Druck eines möglicherweise darin befindlichen Einwegbeutels im befüllten Zustand gut abgefangen wird. D.h. für eine medizinische Behandlung wie Hämodialyse oder Peritonealdialyse wird der Einwegbeutel in die vorzugsweise in der Stützapparatur vorhandene formgebende Schale eingelegt.

Beim Befülllen mit gebrauchsfertigem Dialysat legt sich der erfindungsgemäße Beutel an die Wandung der geformten Stützapparatur an und wird von dieser sicher umschlossen und gestützt. Diese Sicherheit ist mit bisherigen Beuteln der "Batch-Dialyse" nicht gewährleistet. Da das im Beutel und Stützapparatur vorgelegte Dialysat als Bestandteil in der Regel Bikarbonat enthält wird durch das mit gelöstem Bikarbonat im Gleichgeweicht stehende gasförmige CO₂ ein Gasdruck auf die Beutelwandung ausgeübt. CO₂ könnte durch das Folienmaterial entweichen und die Zusammensetzung des Dialysats wäre nicht konstant. Dies wird wirksam durch das Anliegen der elastischen Beutelwandung an die Schale der Stützapparatur verhindert. Die Stützapparatur übernimmt somit zusätzlich die Funktion der Gasbarriere in der systematischen Anordnung aus erfindungsgemäßem Beutel und Stützapparatur.

In einer weiteren bevorzugten Ausführungsform wird der erfindungsgemäße Einwegbeutel am oberen und/oder unteren Ende mit einer Haltevorrichtung, bevorzugt in Form einer Halteschiene versehen, über die er in der Apparatur befestigt werden kann.

Mit Hilfe der Haltevorrichtung kann der Einwegbeutel so in die Apparatur eingebracht und auch wieder entfernt werden. Dabei ist es besonders bevorzugt, dass auch die Zuführ- und/oder Ablassleitung über die Haltevorrichtung befestigt werden.

Die Haltevorrichtung kann entweder fest mit dem Einwegbeutel verbunden sein oder abnehmbar angebracht werden. Im ersten Fall handelt es sich bei der Haltevorrichtung ebenfalls um einen Artikel, der zusammen mit dem erfindungsgemäßen Einwegbeutel entsorgt wird.

Dabei ist es im Sinne der vorliegenden Erfindung weiter möglich, dass mehr als einer der erfindungsgemäßen Einwegbeutel in einer Haltevorrichtung angebracht wird oder aber auch, dass mehrere der erfindungsgemäßen Einwegbeutel in jeweils einer Haltevorrichtung in einer Apparatur angebracht werden.

Weiter betrifft die vorliegende Erfindung die Verwendung des erfindungsgemäßen Einwegbeutels oder der erfindungsgemäßen Vorrichtung zum Einsatz in einer Apparatur zur Durchführung einer medizinischen Behandlung.

In einer weiteren Ausführungsform der vorliegenden Erfindung ist die im letzten Abschnitt genannte Apparatur eine Apparatur zur Durchführung von Hämodialyse oder Peritonealdialyse, d.h. die medizinische Behandlung ist Hämodialyse oder Peritonealdialyse.

Weiter betrifft die vorliegende Erfindung ein Verfahren zur Herstellung des erfindungsgemäßen Einwegbeutels. Das Verfahren umfasst die folgenden Schritte:
a) des Herstellens einer Mehrschichtfolie wie vorstehend definiert; und
b) des Verschweißens der Mehrschichtfolie zu einem Einwegbeutel.

In einer bevorzugten Ausführungsform umfasst das erfindungsgemäße Verfahren zudem den Schritt des Zerteilens der Mehrschichtfolie in mehrere Teile. Dieser Schritt wird bevorzugt vor Schritt b) des vorstehend definierten Verfahrens ausgeführt.

Das erfindungsgemäße Verfahren zur Herstellung einer erfindungsgemäßen Mehrschichtfolie zeichnet sich dadurch aus, dass die Mehrschichtfolie bevorzugt durch Co-Extrusion hergestellt wird. Zur Herstellung der erfindungsgemäßen Mehrschichtfolie werden die Ausgangsmaterialien beispielsweise gemischt, compoundiert und granuliert, bevor sie vorzugsweise auf einer Blasfolienanlage mit Wasserkühlung co-extrudiert werden.

Es ist dabei bevorzugt, dass die Schichten der Mehrschichtfolie in direktem Kontakt zueinander co-extrudiert werden. Es liegt dabei ebenfalls im Rahmen der vorliegenden Erfindung, dass auf einer oder auf beiden Seiten der Mehrschichtfolie eine durch Co-Extrusion erzeugte Schutzfolie angebracht wird. Diese Schutzfolie(n) ist/sind von dem erfindungsgemäßen Mehrschichtfolie leicht und rückstandsfrei zu entfernen bzw. abziehbar.

Desweiteren kann die Mehrschichtfolie jedoch auch in einem separaten Extrusionsverfahren, einem Kalandrierverfahren oder in einem Folienguß-Verfahren erzeugt werden. Der Co-Extrusions-Verband kann zur Erzeugung einer glatten Oberfläche direkt nach dem Extrudieren geglättet werden. Sofern eine Glättung erwünscht ist, kann die Mehrschichtfolie jedoch auch durch jedes andere, dem Fachmann als geeignet bekannte Verfahren geglättet werden.

Die Erfindung ist anhand der nachstehend bevorzugten Ausführungsformen im Zusammenhang mit den Figuren näher beschrieben, was allein der Erläuterung und Veranschaulichung der Erfindung dient und den Rahmen der Erfindung weder einschränken noch begrenzen soll.

Es zeigen:
- Fig. 1: einen erfindungsgemäßen Einwegbeutel mit 2 Kammern mit je einer Zuführ- und einer Ablassleitung
- Fig. 2: einen erfindungsgemäßen Einwegbeutel mit einer Haltevorrichtung in Form einer Halteschiene.
- Fig. 3: einen erfindungsgemäßen Einwegbeutel mit 2 Haltevorrichtungen in Form von Halteschienen.
- Fig. 4: ein Kraft [N] /Dehnung [%] Diagramm der Mehrschichtfolie des erfindungsgemäßen Einwegbeutels
- Fig. 5: ein Kraft [N] /Dehnung [%] Diagramm der Vergleichsfolie 1.
- Fig. 6: ein Kraft [N] /Dehnung [%] Diagramm der Vergleichsfolie 2

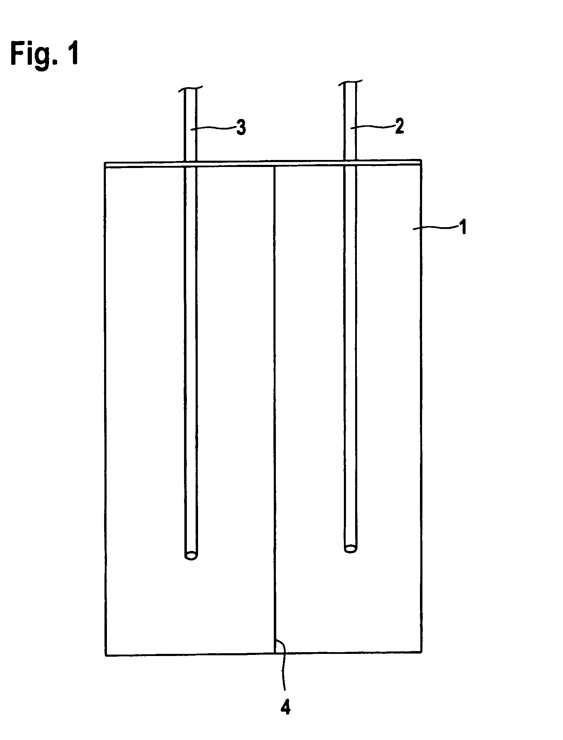

Figur 1 zeigt einen erfindungsgemäßen Einwegbeutel (1). Der erfindungsgemäße Einwegbeutel (1) ist in Seitenansicht dargestellt. Der Einwegbeutel ist durch eine Trennwand/Trennfolie (4) in 2 Kammern unterteilt, die jeweils mit einer Zuführ- oder Ablassleitung ((2), (3)) versehen sind.

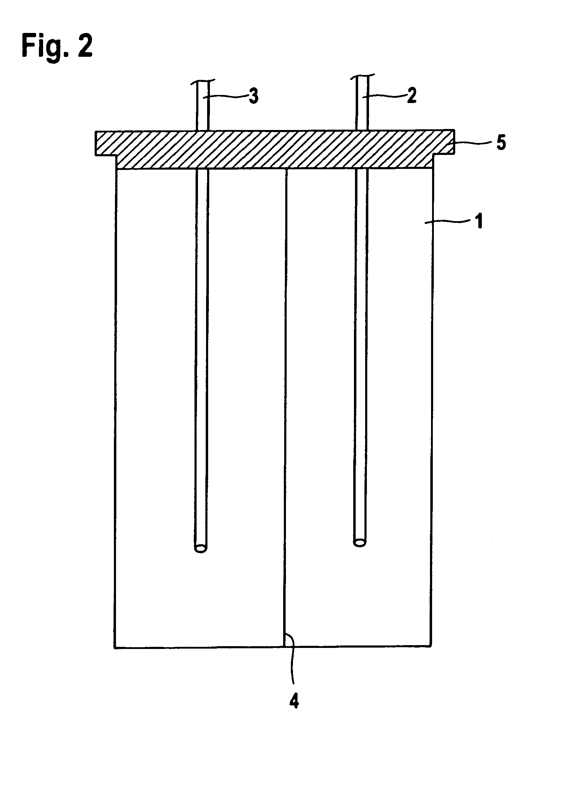

Figur 2 zeigt den erfindungsgemäßen Einwegbeutel der Figur 1, der zudem an der Oberseite, an der auch die Zuführ- und Ablassleitung ((2), (3)) angebracht sind, mit einer Haltevorrichtung (5) in Form einer Halteschiene ausgestattet ist. Die Halteschiene (5) kann hierbei auch dazu dienen, die Zuführ- und Ablassleitung ((2), (3)) zu fixieren.

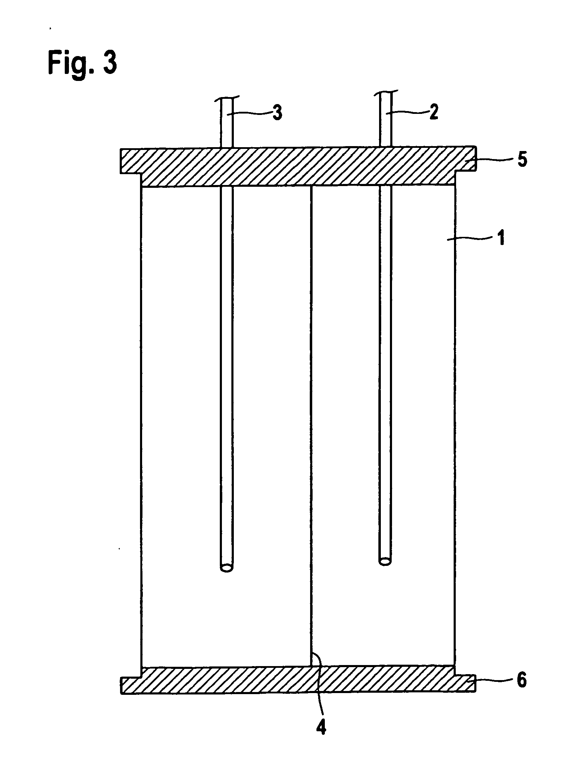

Figur 3 zeigt den erfindungsgemäßen Einwegbeutel aus Figur 2, der zudem auch an der Unterseite mit einer Haltevorrichtung (6) in Form einer Halteschiene ausgestattet ist.

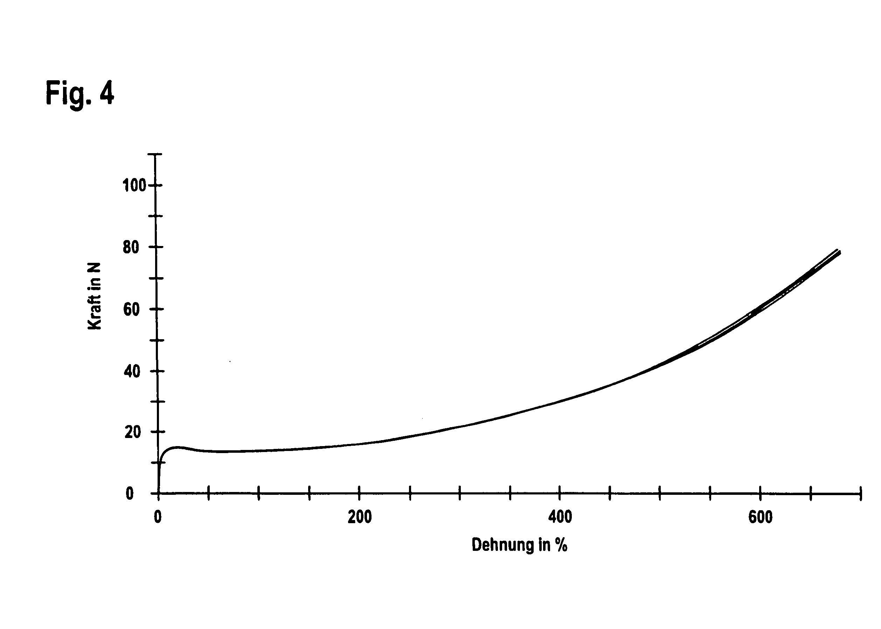

In Figur 4 zeigt ein Kraft[N]/Dehnungs[%]Diagramm der Mehrschichtfolie eines erfindungsgemäßen Einwegbeutels.

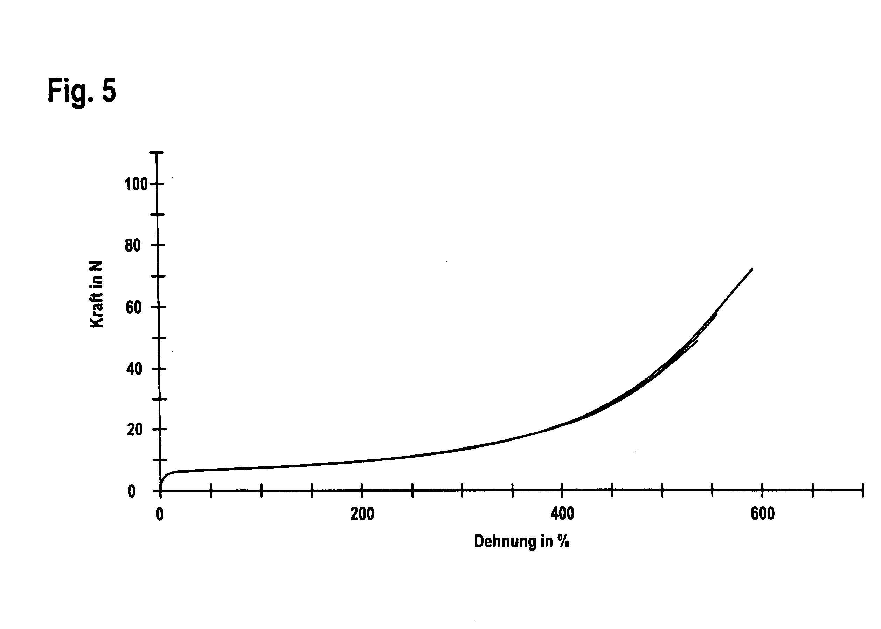

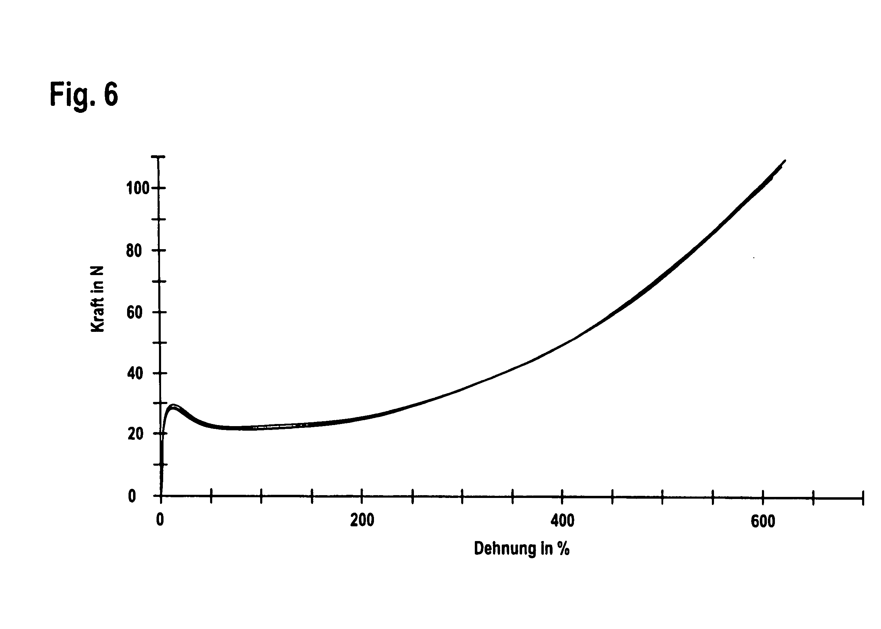

In den Figuren 5 und 6 ist ein Kraft [N]/Dehnungs[%] Diagramm der Vergleichsfolien 1 (Fig. 5) und 2 (Fig. 6) gezeigt.

### Beispiele

In den nachfolgenden Beispielen werden die Vorteile der für den erfindungsgemäßen Einwegbeutel verwendeten Mehrschichtfolie verdeutlicht. Es wird darauf verwiesen, dass die Beispiele und die in den Beispielen genannten Zusammensetzungen der Mehrschichtfolie lediglich verdeutlichenden Charakter besitzen und den Rahmen der Erfindung in keiner Weise einschränken.

Die in den Ausführungsbeispielen 1 bis 5 genannten Muster 1-3 weisen den folgenden Aufbau auf:

### Muster 1

Muster 1 ist eine Dreischichtfolie mit dem folgenden Aufbau:

| | |
|---|---|
| Compound A: | 10 zum |
| Compound B: | 130 µm |
| Compound A: | 10 µm |

Die Außenschichten, wie auch die charakteristische Schicht der Mehrschichtfolie gemäß Muster 1 enthält ein Styrol-Ethylen-Propylen (SEP) Block-Copolymer.

Die folgende Aufstellung zeigt die Zusammensetzung von Muster 1:
Compound A: 100 Teile SEP (Septon 2005; Fa. Kuraray) 70 Teile PP-R Co (PP 23M 10cs264; Fa.Rexene)
Compound B: 100 Teile SEP (Septon 2005; Fa. Kuraray)100 Teile PE-Copolymer (Engage; Fa. DOW)
   (p < 0,9 g/cm³) 70 Teile PP-R Co (PP 23M 10cs264; Fa.Rexene)

Die Abkürzungen bedeuten:
- SEP:: Styrol-Ethylen-Propylen Block-Copolymer
- PP-R Co:: Polypropylen Random-Copolymer
- PP:: Polypropylen
- PE:: Polyethylen.

### Muster 2

Als Muster 2 wurde ebenfalls eine Dreischichtfolie eingesetzt, die wie folgt aufgebaut ist:
Compound A' 10 µm
Compound B' 130 µm
Compound A' 10 µm

Die folgende Aufstellung zeigt die Zusammensetzung von Muster 2:
Compound A: 100 Teile SEB (Molekulargewicht < 120.000 g/mol) 70 Teile PP-R Co (PP 23M 10cs264; Fa.Rexene)
Compound B: 100 Teile SEB (Molekulargewicht < 120.000 g/mol)100 Teile PE-Copolymer (Engage; Fa. DOW) (p < 0,9 g / cm³)

Die Abkürzungen bedeuten:
SEB: Styrol-Ethylen-Butylen Block-Copolymer
PP-R Co: Polypropylen Random-Copolymer
PE: Polyethylen.

### Muster 3

Die Folie gemäß Muster 3 ist eine kommerziell erhältliche Folie der Firma Nissho. Es handelt sich dabei um eine Monofolie mit der folgenden Zusammensetzung:
54% SEB
23-35% PP
5-23% EAA.

Die Abkürzungen bedeuten:
SEB: Styrol-Ethylen-Butylen Block-Copolymer
PP: Polypropylen Homopolymer
EAA: Ethylen-Acrylsäure-Polymer.

### Beispiel 1

### Verformung bei Hitzesterilisation

Um die Verformung der Folien bei der Hitzesterilisation bei einer Temperatur von 121 °C zu untersuchen, wurde jeweils ein 1,33 dm² großes Folienstück spannungsfrei für 40 Minuten hitzesterilisiert. Dabei ergaben sich die folgenden Resultate: Schrumpfen in Längsrichtung: Muster 1: 3,2 %, Muster 2: 11,1 %, Muster 3: 0,4 %*Schrumpfen in Querrichtung: Muster 1: 1,6 %, Muster 2: 2,1 %, Muster 3: 0,4 %*(* Folie nach Muster 3 war zuvor bereits ETO-sterilisiert (Ethylenoxid-sterilisiert) und damit vermutlich spannungsfrei.)

### Beispiel 2

### Verkleben bei Hitzesterilisation

Um das Verkleben der Folien bei der Hitzesterilisation zu untersuchen, wurden aus entsprechenden Folien hergestellte leere Beutel bei einer Temperatur von 121 °C für 40 Minuten hitzesterilisiert. Dabei zeigten die Beutelinnenflächen bei Verwendung der Mehrschichtfolie nach Muster 1 kein messbares Haften oder Verkleben. Im Gegensatz dazu waren die Beutelinnenflächen bei Verwendung der Mehrschichtfolie gemäß Muster 2 nach der Hitzesterilisation verklebt und konnten erst durch einen hydrostatischen Druck von ca. 320 mm WS (Wassersäule) innerhalb von ca. 90 Sekunden auf einer Länge von 10 cm gelöst werden. Bei der Verwendung von Muster 3 erhielt man nach der Hitzesterilisation deutlich miteinander verklebte Beutelinnenflächen, die sich auch nach einer zehnminütigen Druckbeaufschlagung mit Wasser bei 300 mm Hg nicht vollständig voneinander lösten.

### Beispiel 3

### Versprödungstemperatur

Die Untersuchungen zur Versprödungstemperatur der Folien wurden nach DIN 53443 T 2 durchgeführt. Dabei zeigte Muster 1 einen beginnenden Sprödbruch bei -60 °C, Muster 2 bei -65 °C und Muster 3 bei -60 °C.

### Beispiel 4

### Lastwechselfestigkeit

Die Versuche zur Bestimmung der Lastwechselfestigkeit erfolgten mit einer pneumatisch angetriebenen Versuchseinrichtung. Dabei wurde die Mehrschichtfolie zwischen zwei Gehäuse-Hälften fixiert. An der Gehäuse-Unterseite lag die Folie eben an, während sich auf der Gehäuse-Oberseite eine 25 ml fassende Kalotte erstreckte. Durch eine wechselseitige Druckbeaufschlagung mit 1,5 bar wurde die Mehrschichtfolie zu Schwingungen zwischen der Kalotte und der ebenen Fläche angeregt. Dabei betrug die theoretische Dehnung bei gleichmäßiger Verformung der Folie bis zu 43%. Die Untersuchungen wurden bei 37 °C mit hitzesterilisierten Folien entsprechend Muster 1 und Muster 2 durchgeführt.

Bei den sich an die Lastwechselversuche anschließenden Untersuchungen durch Lichtmikroskopie, REM und Luftleckage gegen Wasser konnten auch nach mehr als 20 000 Lastwechseln weder Risse noch Löcher in den Probenstücken der Mehrschichtfolien festgestellt werden. Die Probenstücke zeigten partiell lediglich eine geringe Faltenbildung, die auf eine geringfügige bleibende Dehnung in diesen Bereichen zurückgeführt werden kann.

### Beispiel 5

### Gasdurchlässigkeit

Die folgende Tabelle 1 zeigt die Gasdurchlässigkeit der Muster 1, 2 und 3 für O₂ und CO₂, wobei die O₂-Gasdurchlässigkeit nach ASTM D-3985, DIN 53380 bestimmt wurde.

**Tabelle 1: Gasdurchlässigkeit**

| Muster | | O₂ cm² m²*d*bar | | CO₂ cm³/M2*d*bar | |
|---|---|---|---|---|---|
| | | 95% r.F-23 °C | | 95%r.F-23 °C | |
| 1 | | 3425 | | 9901 | |
| 2 | | 3021 | | 9628 | |
| 3 | | 1654 | | 4047 | |

Aus Beispiel 5 ist ersichtlich, dass die Gasdurchlässigkeit der erfindungsgemäßen Folie nach Muster 1 sowohl für O₂ als auch für CO₂ deutlich insbesondere über der kommerziell erhältlichen Folie nach Muster 3 liegt.

Beispiel 2 zeigt darüber hinaus, dass die erfindungsgemäße Mehrschichtfolie nach Muster 1 bei der Hitzesterilisation nicht messbar zum Verkleben neigt, was darauf zurückzuführen ist, dass die außenliegenden Schichten aus mindestens 55 % hitzesterilisierbarem Styrol-Ethylen-Propylen Block-Copolymer mit einem mittleren Molekulargewicht ≥ 120 000 g/mol und weniger als 45 % Polypropylen bestehen.

### Beispiel 6

Mit einer Probe der Mehrschichtfolie des erfindungsgemäßen Einwegbeutels wurde ein Zugversuch durchgeführt. Getestet wurden Proben mit einer Breite von 15 mm sowie einer Dicke von 120 µm. Der Versuch wurde sowohl mit Proben durchgeführt, die quer zur Extrusionsrichtung (CD) als auch längs zur Extrusionsrichtung (MD) ausgestanzt wurden. Der Aufbau der Mehrschichtfolie war dabei wie folgt:
- PP/SEBS 8 µm (40 % RD 204 CF; Fa. Borealis; 60 % Septon 8004, Fa. Kuraray)
- PE/SEBS 97 µm (25 % Tuftec H1062, Fa. Asahi; 40 % Engage 8003, Fa. Dow; 35 % Septon 8004, Fa. Kuraray)
- PP/SEBS 15 µm (40 % RD 204 CF, Fa. Borealis; 60 % Septon 8004, Fa. Kuraray).

Die Ergebnisse werden in der nachfolgenden Tabelle 2 gezeigt:

**Tabelle 2: Ergebnisse des Zugversuches**

| MPa | > 50 N /15 mm | > 500 % | MPa | > 50 N /15 mm | > 550 % |
|---|---|---|---|---|---|
| E-Modul MD | Zugfestigkeit MD | Zugdehnung MD | E-Modul CD | Zugfestigkeit CD | Zugdehnung CD |
| 58 | 62,4 | 579, 4 | 21 | 62, 7 | 628 |

Der Zugversuch wurde gemäß DIN EN 527 1-3 bei einer Vorschubgeschwindigkeit von 1 mm/min durchgeführt.

Vergleichsversuche wurden mit aus dem Stand der Technik bekannten Folien durchgeführt.

Getestet wurden ebenfalls Proben mit einer Breite von 15 mm sowie einer Dicke von 200 µm (Vergleichsfolie 1) und 120 µm (Vergleichsfolie 2).Der Aufbau der Vergleichsfolien war dabei wie folgt:

### Vergleichsfolie 1:

| | |
|---|---|
| Aufbau | ca.200 µm Dicke |
| Außen ca.15 µm | 100 % Polypropylen-Ethylen Copolymer |
| Mitte ca.160 µm | 35 % SEBS, 65 % Polypropylen-Ethylen Copolymer |
| Innen ca.20 µm | 80 % Polypropylen-Ethylen Copolymer, 20% SEBS |

### Vergleichsfolie 2:

| | |
|---|---|
| Aufbau | ca.180 µm Dicke |
| Außen ca.20 µm | 100 % Polypropylen-Ethylen Copolymer |
| Mitte ca.120 µm | 60 % SEBS, 40 % Polypropylen-Ethylen Copolymer |
| Innen ca.20 µm | 100 % Polypropylen-Ethylen Copolymer |

Die Ergebnisse werden in den nachfolgenden Tabellen 3 (Vergleichsfolie 1) und 4 (Vergleichsfolie 2) gezeigt:

**Tabelle 3: Vergleichsfolie 1**

| MPa | >12N/ 15mm | >65N/ 15mm | 1>8% | >550% | MPa | >10N/ 15mm | >55N/ 15mm | >8% | >500% | >20N/ 15mm | 3-8N/15 mm |
|---|---|---|---|---|---|---|---|---|---|---|---|
| E-Modul MD | Str.-sp. MD | Zugfest. MD | Str. dehn MD | Zugdehn. MD | E-Modul CD | Str.-sp. CD | Zugfest. CD | Str. dehn CD | Zugdehn. CD | Schwei ssnaht | Peeln aht |
| 254 | 30,1 | 114,7 | 10,9 | 631 | 180 | 23, 9 | 112,6 | 13, 4 | 640 | 44, 3 | 4,9 |

**Tabelle 4: Vergleichsfolie 2**

| MPa | >55N/15mm | >500% | MPa | >55N/15mm | >500% | >20N/15mm |
|---|---|---|---|---|---|---|
| E-Modul MD | Zugfest. MD | Zugdehn. MD | E -Modul CD | Zugfest. CD | Zugdehn CD | Schweissnaht |
| 86, 9 | 79,8 | 665,9 | 80,7 | 78,3 | 661,5 | 28,5 |

## Patentansprüche

1. Elastisch dehnbarer Einwegbeutel aus einer Mehrschichtfolie zur Aufnahme einer Dialysierflüssigkeit, wobei die Mehrschichtfolie in Längsrichtung eine Reißdehnung von 250 % bis 850 % und in Querrichtung von 300 % bis 1050 % aufweist;
wobei die Mehrschichtfolie eine Schicht des Typs (A) umfasst, die mehrere Komponenten enthält, wobei eine der Komponenten aus der Gruppe ausgewählt ist, die aus Styrol-Isopren Block-Copolymeren, Styrol-Ethylen-Butylen Block-Copolymeren, Styrol-Ethylen-Propylen Block-Copolymeren und Mischungen davon besteht, und eine weitere Komponente aus der Gruppe ausgewählt ist, die aus Polyethylen und einem Copolymer, das Ethylen-Einheiten umfasst, besteht;
wobei die Mehrschichtfolie zudem eine oder mehrere Schichte(n) des Typs (B) umfasst, die unabhängig voneinander mehrere Komponenten enthält, wobei eine der Komponenten aus der Gruppe ausgewählt ist, die aus Styrol-Ethylen-Butylen Block-Copolymere, Styrol-Ethylen-Propylen Block-Copolymere, Styrol-Isopren Block-Copolymere und Mischungen davon besteht, und eine weitere Komponente aus der Gruppe ausgewählt ist, die aus Polypropylen und einem Copolymer, das Propylen-Einheiten umfasst, besteht; und wobei das Fassungsvolumen des Einwegbeutels im maximal befüllten Zustand zu dem Fassungsvolumen im Zustand, in dem die Mehrschichtfolie dehnungsfrei vorliegt, ≥ 3/1 ist.

2. Einwegbeutel gemäß Anspruch 1, wobei die Mehrschichtfolie in Längsrichtung eine Reißfestigkeit von 300 kp/cm² bis 350 kp/cm² und in Querrichtung von 220 kp/cm² bis 270 kp/cm² aufweist.

3. Einwegbeutel gemäß Anspruch 1 oder 2, wobei die Mehrschichtfolie eine Querdehnzahl µ im gummielastischen Zustand von 0,45 bis 0,55 aufweist.

4. Einwegbeutel gemäß einem der Ansprüche 1 bis 3, wobei die Mehrschichtfolie durch eine Kraft von 45 N/ 15 mm bis 60 N/ 15 mm um bis zu 500 % gedehnt werden kann.

5. Einwegbeutel gemäß einem der Ansprüche 1 bis 4, wobei das Verhältnis der äußeren Oberfläche des Einwegbeutels im maximal befüllten Zustand zu der äußeren Oberfläche im unbefüllten Zustand ≥2/1 ist.

6. Einwegbeutel gemäß einem der Ansprüche 1 bis 5, wobei das Fassungsvolumen des Einwegbeutels im maximal befüllten Zustand im Bereich von 30 L bis 120 L liegt.

7. Einwegbeutel gemäß einem der Ansprüche 1 bis 6, der mehrere Kammern enthält.

8. Einwegbeutel gemäß Anspruch 7, wobei die Kammern nebeneinander in Längsrichtung des Einwegbeutels angeordnet sind.

9. Einwegbeutel gemäß einem der Ansprüche 1 bis 8, der weiter eine Zuführ- und eine Ablassleitung aufweist.

10. Einwegbeutel gemäß Anspruch 9, wobei Zuführ- und Ablassleitung an verschiedenen Kammern angebracht sind.

11. Einwegbeutel gemäß Anspruch 1, wobei der Anteil der weiteren Komponente in der Schicht des Typs (A) im Bereich von 0,01 % bis 65 % liegt, bezogen auf die gesamte Zusammensetzung der Schicht des Typs (A).

12. Einwegbeutel gemäß Anspruch 1, wobei die erste Komponente in der Schicht des Typs (B) ein mittleres Molekulargewicht von ≥ 120000 g/mol aufweist.

13. Einwegbeutel gemäß Anspruch 1 oder 12, **dadurch gekennzeichnet, dass** die Schicht des Typs (A) eine mittlere Schicht ist, die von zwei weiteren Schichten des Typs (B) umgeben ist.

14. Vorrichtung, die eine Stützapparatur umfasst, in der ein Einwegbeutel gemäß einem der Ansprüche 1 bis 13 eingebracht ist.

15. Vorrichtung gemäß Anspruch 14, **dadurch gekennzeichnet, dass** die Vorrichtung weiter eine Haltevorrichtung umfasst, an der der Einwegbeutel befestigt ist.

16. Vorrichtung gemäß Anspruch 14 oder 15, **dadurch gekennzeichnet, dass** die Stützapparatur eine formgebende Schale umfasst, die mit dem Einwegbeutel in Verbindung ist.

17. Verwendung eines Einwegbeutels der Ansprüche 1 bis 13 oder der Vorrichtung gemäß den Ansprüchen 14 bis 16 zum Einsatz in einer Apparatur zur Durchführung einer medizinischen Behandlung.

18. Verwendung gemäß Anspruch 17, wobei die Apparatur eine Apparatur zur Durchführung von Hämodialyse oder Peritonealdialyse ist.

19. Verfahren zur Herstellung eines Einwegbeutels gemäß einem der Ansprüche 1 bis 13, umfassend die folgenden Schritte des:
a) Herstellens einer Mehrschichtfolie wie in einem der Ansprüche 1 bis 4 und 11 bis 13 definiert;
b) Verschweißens der Mehrschichtfolie zu einem Beutel.

## Claims

1. Elastically extensible disposable bag comprising a multilayer film for accommodating a dialysis fluid, wherein the multilayer film has a tensile elongation in longitudinal direction of from 250% to 850%, and in transverse direction of from 300% to 1050%;
wherein the multilayer film comprises a type (A) layer which contains several components, wherein one of the components is selected from the group which consists of styrene-isoprene block copolymers, styrene-ethylene-butylene block copolymers, styrene-ethylene-propylene block copolymers and mixtures thereof, and a further component is selected from the group which consists of polyethylene and a copolymer which comprises ethylene units;
wherein the multilayer film also comprises one or more type (B) layer(s) which contains several components independently of one another, wherein one of the components is selected from the group which consists of styrene-ethylene-butylene block copolymers, styrene-ethylene-propylene block copolymers, styrene-isoprene block copolymers and mixtures thereof, and a further component is selected from the group which consists of polypropylene and a copolymer which comprises propylene units; and
wherein the holding capacity of the disposable bag when filled to the maximum relative to the holding capacity in the state in which the multilayer film is present unextended is ≥ 3/1.

2. Disposable bag according to claim 1, wherein the multilayer film has a tear strength in longitudinal direction of from 300 kp/cm² to 350 kp/cm², and in transverse direction of from 220 kp/cm² to 270 kp/cm².

3. Disposable bag according to claim 1 or 2, wherein the multilayer film has a transverse strain ratio µ in the rubber-elastic state of from 0.45 to 0.55.

4. Disposable bag according to one of claims 1 to 3, wherein the multilayer film can be extended by up to 500% by a force of from 45 N/ 15 mm to 60 N/ 15 mm.

5. Disposable bag according to one of claims 1 to 4, wherein the ratio of the external surface of the disposable bag when filled to the maximum to the external surface when unfilled is ≥2/1.

6. Disposable bag according to one of claims 1 to 5, wherein the holding capacity of the disposable bag when filled to the maximum lies in the range of from 30 L to 120 L.

7. Disposable bag according to one of claims 1 to 6, which contains several chambers.

8. Disposable bag according to claim 7, wherein the chambers are arranged alongside one another in longitudinal direction of the disposable bag.

9. Disposable bag according to one of claims 1 to 8, which also has a feed line and a drainage line.

10. Disposable bag according to claim 9, wherein feed line and drainage line are attached to different chambers.

11. Disposable bag according to claim 1, wherein the proportion of the further component in the type (A) layer lies in the range of from 0.01% to 65%, relative to the total composition of the type (A) layer.

12. Disposable bag according to claim 1, wherein the first component in the type (B) layer has an average molecular weight of ≥120000 g/mol.

13. Disposable bag according to claim 1 or 12, **characterized in that** the type (A) layer is a middle layer which is surrounded by two further type (B) layers.

14. Device, which comprises a support apparatus in which a disposable bag according to one of claims 1 to 13 is introduced.

15. Device according to claim 14, **characterized in that** the device also comprises a holder to which the disposable bag is fixed.

16. Device according to claim 14 or 15, **characterized in that** the support apparatus comprises a shaping dish which is connected to the disposable bag.

17. Use of a disposable bag of claims 1 to 13 or of the device according to claims 14 to 16, for use in an apparatus for carrying out a medical treatment.

18. Use according to claim 17, wherein the apparatus is an apparatus for carrying out haemodialysis or peritoneal dialysis.

19. Process for producing a disposable bag according to one of claims 1 to 13, comprising the following steps:
a) producing a multilayer film as defined in one of claims 1 to 4 and 11 to 13;
b) welding the multilayer film into a bag.

## Revendications

1. Sachet à usage unique étirable élastiquement constitué d'un film multicouches pour la réception d'un liquide de dialyse, dans lequel le film multicouches présente une élongation à la rupture de 250 % à 850 % dans la direction longitudinale et de 300 % à 1050 % dans la direction transversale ;
dans lequel le film multicouches comprend une couche du type (A) qui contient plusieurs composants, dans lequel un des composants est sélectionné parmi le groupe qui se compose de copolymères à blocs de styrène-isoprène, de copolymères à blocs de styrène-éthylène-butylène, de copolymères à blocs de styrène-éthylène-propylène et de mélanges de ceux-ci, et un autre composant est sélectionné parmi le groupe qui se compose de polyéthylène et d'un copolymère qui comprend des fragments d'éthylène ;
dans lequel le film multicouches comprend en outre une ou plusieurs couche(s) du type (B) qui contient plusieurs composants indépendamment les uns des autres, dans lequel un des composants est sélectionné parmi le groupe qui se compose de copolymères à blocs de styrène-éthylène-butylène, de copolymères à blocs de styrène-éthylène-propylène, de copolymères à blocs de styrène-isoprène et de mélanges de ceux-ci, et un autre composant est sélectionné parmi le groupe qui se compose de polypropylène et d'un copolymère qui comprend des fragments de propylène ; et
dans lequel la capacité du sachet à usage unique dans l'état rempli au maximum par rapport à la capacité dans l'état dans lequel le film multicouches est présent de manière exempte d'élongation est ≥3/1.

2. Sachet à usage unique selon la revendication 1, dans lequel le film multicouches présente une résistance à la rupture de 300 kp/cm² à 350 kp/cm² dans la direction longitudinale et de 220 kp/cm² à 270 kp/cm² dans la direction transversale.

3. Sachet à usage unique selon la revendication 1 ou 2, dans lequel le film multicouches présente un indice d'élongation transversale dans l'état élastique comme du caoutchouc de 0,45 à 0,55.

4. Sachet à usage unique selon l'une quelconque des revendications 1 à 3, dans lequel le film multicouches peut être étiré jusqu'à 500 % par une force de 45 N/15 mm à 60 N/15 mm.

5. Sachet à usage unique selon l'une quelconque des revendications 1 à 4, dans lequel le rapport entre la surface extérieure du sachet à usage unique dans l'état rempli au maximum et la surface extérieure dans l'état non rempli est ≥2/1.

6. Sachet à usage unique selon l'une quelconque des revendications 1 à 5, dans lequel la capacité du sachet à usage unique dans l'état rempli au maximum se situe dans la plage de 30 1 à 120 1.

7. Sachet à usage unique selon l'une quelconque des revendications 1 à 6, qui contient plusieurs chambres.

8. Sachet à usage unique selon la revendication 7, dans lequel les chambres sont disposées les unes à côté des autres dans la direction longitudinale du sachet à usage unique.

9. Sachet à usage unique selon l'une quelconque des revendications 1 à 8, qui présente en outre une conduite d'alimentation et une conduite d'évacuation.

10. Sachet à usage unique selon la revendication 9, dans lequel les conduites d'alimentation et d'évacuation sont appliquées sur différentes chambres.

11. Sachet à usage unique selon la revendication 1, dans lequel la proportion de l'autre composant dans la couche du type (A) se situe dans la plage de 0,01 % à 65 %, par rapport à la composition totale de la couche du type (A).

12. Sachet à usage unique selon la revendication 1, dans lequel le premier composant dans la couche du type (B) présente un poids moléculaire moyen
≥ 120 000 g/mol.

13. Sachet à usage unique selon la revendication 1 ou 12, **caractérisé en ce que** la couche du type (A) est une couche centrale qui est entourée par deux autres couches du type (B).

14. Dispositif qui comprend un appareil de support dans lequel un sachet à usage unique selon l'une quelconque des revendications 1 à 13 est introduit.

15. Dispositif selon la revendication 14, **caractérisé en ce que** le dispositif comprend en outre un dispositif de maintien auquel le sachet à usage unique est fixé.

16. Dispositif selon la revendication 14 ou 15, **caractérisé en ce que** le dispositif de support comprend une coque de modelage qui est connectée au sachet à usage unique.

17. Utilisation d'un sachet à usage unique selon les revendications 1 à 13 ou du dispositif selon les revendications 14 à 16 pour l'insertion dans un appareil en vue de la réalisation d'un traitement médical.

18. Utilisation selon la revendication 17, dans laquelle l'appareil est un appareil pour la réalisation d'une hémodialyse ou d'une dialyse péritonéale.

19. Procédé de fabrication d'un sachet à usage unique selon l'une quelconque des revendications 1 à 13, comprenant les étapes suivantes consistant à :
a) fabriquer un film multicouches comme défini dans une des revendications 1 à 4 et 11 à 13 ;
b) souder le film multicouches à un sachet.
